(19) Europäisches Patentamt
European Patent Office
Office européen des brevets

(11) Veröffentlichungsnummer: **0 290 003**
**A2**

(12) **EUROPÄISCHE PATENTANMELDUNG**

(21) Anmeldenummer: **88107138.5**

(22) Anmeldetag: **04.05.88**

(51) Int. Cl.⁴: **C07D 471/04 , C07D 487/04 , A61K 31/435 , A61K 31/495 , //(C07D471/04,235:00,221:00), (C07D487/04,241:00,235:00)**

(30) Priorität: **08.05.87 CH 1771/87**

(43) Veröffentlichungstag der Anmeldung: **09.11.88 Patentblatt 88/45**

(84) Benannte Vertragsstaaten: **ES GR**

(71) Anmelder: **Byk Gulden Lomberg Chemische Fabrik GmbH**
**Byk-Gulden-Strasse 2**
**D-7750 Konstanz(DE)**

(72) Erfinder: **Senn-Bilfinger, Dr.**
**Säntisstrasse 7**
**D-7750 Konstanz(DE)**
Erfinder: **Grundler, Gerhard, Dr.**
**Meersburger Strasse 4**
**D-7750 Konstanz(DE)**
Erfinder: **Schaefer, Hartmann, Dr.**
**Oberdorfstrasse 4**
**D-7750 Konstanz 18(DE)**
Erfinder: **Klemm, Kurt, Prof. Dr.**
**Im Weinberg 2**
**D-7753 Allensbach(DE)**
Erfinder: **Rainer, Georg, Dr.**
**Josef-Anton-Feuchtmayer-Strasse 7**
**D-7750 Konstanz(DE)**
Erfinder: **Riedel, Richard, Dr.**
**Salmannsweilergasse 36**
**D-7750 Konstanz(DE)**
Erfinder: **Schudt, Christian, Dr.**
**Hoheneggstrasse 102**
**D-7750 Konstanz(DE)**
Erfinder: **Simon, Wolfgang, Dr.**
**Seestrasse 31a**
**D-7750 Konstanz(DE)**

(54) **Neue Imidazole.**

(57) Verbindungen der Formel I,

EP 0 290 003 A2

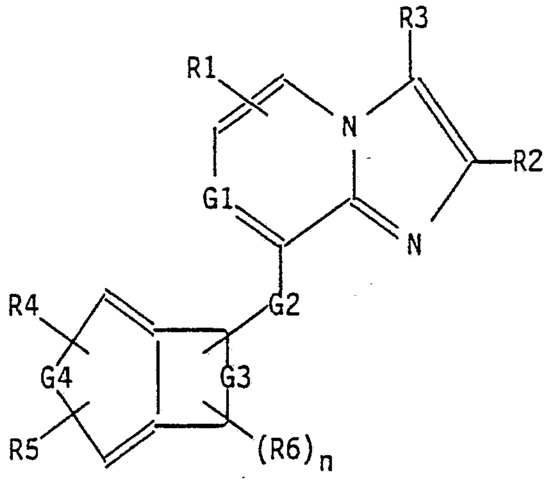

(I)

worin die Substituenten und Symbole die in der Beschreibung genannten Bedeutungen haben, sind neue Verbindungen mit interessanten pharmakologischen Eigenschaften.

# Neue Imidazole

## Anwendungsgebiet der Erfindung

Die Erfindung betrifft neue Imidazole, Verfahren zu ihrer Herstellung, ihre Anwendung und sie enthaltende Arzneimittel. Die erfindungsgemäßen Verbindungen werden in der pharmazeutischen Industrie als Zwischenprodukte und zur Herstellung von Medikamenten verwendet.

## Bekannter technischer Hintergrund

Aus den veröffentlichten Europäischen Patentanmeldungen 0 033 094, 0 068 378, 0 165 545 und 0 204 285 sind Imidazopyridine bzw. Imidazoisochinoline bekannt, die aufgrund ihrer antisekretorischen und cytoprotektiven Wirkung bei der Behandlung von Ulcuserkrankungen eingesetzt werden sollen.

## Beschriebung der Erfindung

Es wurde nun gefunden, daß die unten näher beschriebenen neuen Imidazolverbindungen interessante pharmakologische Eigenschaften aufweisen, durch die sie sich von den obenerwähten bekannten Verbindungen in überraschender und besonders vorteilhafter Weise unterscheiden.

Gegenstand der Erfindung sind neue Imidazolverbindungen der Formel I

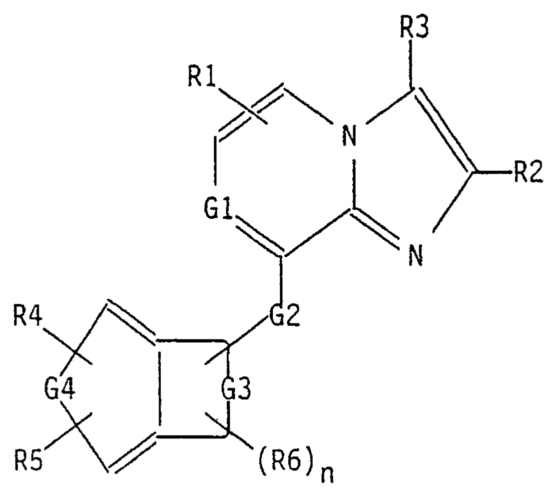

(I)

worin

R1 Wasserstoff (H), Halogen, 1-4C-Alkyl, 1-4C-Alkoxy, 1-4C-Alkoxycarbonyl oder Cyan,
R2 Wasserstoff (H), 1-4C-Alkyl-Hydroxy-1-4C-alkyl, Halo-1-4C-alkyl, Cyano-1-4C-alkyl oder 1-4C-Alkoxycarbonyl,
R3 Wasserstoff (H), 1-4C-Alkyl, Formyl, Hydroxy-1-4C-alkyl, Halo-1-4C-alkyl, Cyano-1-4C-alkyl, Amino-1-4C-alkyl, Mono-oder Di-1-4C-alkylamino-1-4C-alkyl, Nitroso, Nitro, Amino, Mono-oder Di-1-4C-alkylamino oder 1-4C-Alkoxycarbonyl,
R4 Wasserstoff (H), 1-4C-Alkyl, 1-4C-Alkoxy, Halogen oder Trifluormethyl,
R5 Wasserstoff (H), 1-4C-Alkyl, 1-4C-Alkoxy, Halogen oder Trifluormethyl,
R6 Wasserstoff oder 1-4C-Alkyl,
n die Zahlen 1 oder 2,
G1 CH oder N (Stickstoff),

G2 O (Sauerstoff), NH, N-1-4C-Alkyl, S (Schwefel), Methin (=CH-) oder 1-4C-Alkylen,
G3 Ethylen ($-CH_2CH_2-$), Trimethylen ($-CH_2-CH_2-CH_2-$), Tetramethylen ($-CH_2CH_2CH_2CH_2-$), Pentamethylen ($-CH_2CH_2CH_2CH_2CH_2-$), Propenylen ($-CH=CH-CH_2-$), 1-Butenylen ($-CH=CH-CH_2-CH_2-$) oder 2-Butenylen ($-CH_2-CH=CH-CH_2-$) und
G4 S (Schwefel), O (Sauerstoff) oder Vinylen ($-CH=CH-$) bedeutet, und ihre Salze.

Halogen im Sinne der vorliegenden Erfindung ist Brom und insbesondere Chlor und Fluor.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl-und insbesondere der Methylrest.

1-4C-Alkoxyreste enthalten neben dem Sauerstoffatom einen der vorstehend genannten 1-4C-Alkylreste. Bevorzugt ist der Methoxyrest.

1-4C-Alkoxycarbonylreste enthalten neben der Carbonyloxygruppe einen der vorstehend genannten 1-4C-Alkylreste. Bevorzugt sind der Methoxycarbonyl-und der Ethoxycarbonylrest.

Hydroxy-1-4C-alkyl steht für die vorstehend genannten 1-4C-Alkylreste, an die ein Hydroxylrest gebunden ist. Bevorzugt ist der Hydroxymethylrest.

Halo-1-4C-alkyl steht für die vorstehend genannten 1-4C-Alkylreste, an die ein Halogenatom gebunden ist. Bevorzugt ist der Chlormethylrest.

Cyano-1-4C-alkyl steht für die vorstehend genannten 1-4C-Alkylreste, an die ein Cyanrest gebunden ist. Bevorzugt ist der Cyanomethylrest.

Amino-1-4C-alkyl steht für die vorstehend genannten 1-4C-Alkylreste, an die eine Aminogruppe gebunden ist.

Mono-oder Di-1-4C-alkylamino steht für Aminogruppen, die durch eine oder zwei der vorstehend genannten 1-4C-Alkylreste substituiert sind. Beispielsweise seien die Methylamino, Ethylamino, Diisopropylamino und insbesondere die Dimethylaminogruppe genannt.

Mono-oder Di-1-4C-alkylamino-1-4C-alkyl steht für die vorstehend genannten 1-4C-Alkylreste, an die eine der vorstehend genannten Mono-oder Di-1-4C-alkyl-aminogruppen gebunden ist. Bevorzugt sind der Dimethylaminomethyl-und der Dimethylaminoethylrest.

1-4C-Alkylen steht für geradkettige Alkylenreste mit 1 bis 4 Kohlenstoffatomen. Als bevorzugte Alkylenreste seien der Ethylenrest ($-CH_2-CH_2-$) und der Methylenrest ($-CH_2-$) genannt.

Als Salze kommen für Verbindungen der Formel I einerseits bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosilat, 2-Hydroxy-3-naphthoat, 3-Hydroxy-2-naphthoat oder Mesilat.

Anderseits kommen als Salze aber auch quartäre Ammoniumsalze in Betracht, die durch die Umsetzung von Verbindungen der Formel I mit geeigneten Alkylierungsmitteln erhalten werden können. Als geeignete Alkylierungsmittel seien beispielsweise 1-4C-Alkylhalogenide, bevorzugt Methyliodid, oder Benzylhalogenide, wie Benzylbromid, oder Allylhalogenide, wie Allylbromid, genannt.

Erwähnenswerte erfindungsgemäße Verbindungen sind solche der Formel I, worin
R1 Wasserstoff, Chlor oder Fluor,
R2 Wasserstoff, Methyl, Ethyl, Hydroxymethyl, Chlormethyl, Cyanomethyl, Methoxycarbonyl oder Ethoxycarbonyl,
R3 Wasserstoff, Methyl, Formyl, Hydroxymethyl, Chlormethyl, Cyanomethyl, Dimethylaminomethyl, Dimethylaminoethyl, Amino, Methoxycarbonyl oder Ethoxycarbonyl,
R4 Wasserstoff, Methyl, Methoxy, Chlor, Fluor oder Trifluormethyl,
R5 Wasserstoff,
R6 Wasserstoff oder Methyl,
n die Zahlen 1 oder 2,
G1 CH oder N (Stickstoff),
G2 O (Sauerstoff), NH, Methin (=CH-) oder Methylen,
G3 Trimethylen ($-CH_2-CH_2-CH_2-$), Tetramethylen ($-CH_2-CH_2-CH_2-CH_2-$), Pentamethylen ($-CH_2CH_2CH_2CH_2CH_2-$), Propenylen ($-CH=CH-CH_2-$), 1-Butenylen ($-CH=CH-CH_2CH_2-$) oder 2-Butenylen ($-CH_2-CH=CH-CH_2-$) und

G4 O (Sauerstoff), S (Schwefel) oder Vinylen (-CH = CH-) bedeutet, und ihre Salze.

Hervorzuhebende erfindungsgemäße Verbindungen sind solche der Formel I, worin
R1 Wasserstoff, Chlor oder Fluor,
R2 Wasserstoff, Methyl oder Hydroxymethyl,
R3 Wasserstoff, Methyl, Formyl, Hydroxymethyl, Chlormethyl, Cyanomethyl oder Dimethylaminomethyl
R4 Wasserstoff, Methyl, Chlor oder Fluor
R5 Wasserstoff,
R6 Wasserstoff
G1 CH oder N (Stickstoff),
G2 O (Sauerstoff), NH, Methin ( = CH-) oder Methylen,
G3 Trimethylen ($-CH_2-CH_2-CH_2-$), Tetramethylen ($-CH_2CH_2CH_2CH_2-$) oder Propenylen ($-CH=CH-CH_2-$) und
G4 O (Sauerstoff), S (Schwefel) oder Vinylen (-CH = CH-) bedeutet, und ihre Salze.

Besonders hervorzuhebende erfindungsgemäße Verbindungen sind solche der Formel I, worin
R1 Wasserstoff,
R2 Methyl,
R3 Wasserstoff, Methyl, Formyl, Hydroxymethyl, Cyanomethyl oder Dimethylaminomethyl,
R4 Wasserstoff,
R5 Wasserstoff,
R6 Wasserstoff,
G1 CH oder N (Stickstoff),
G2 O (Sauerstoff), NH, Methin ( = CH-) oder Methylen,
G3 Trimethylen ($-CH_2-CH_2-CH_2-$), Tetramethylen ($-CH_2-CH_2-CH_2-CH_2-$), oder Propenylen ($-CH=CH-CH_2-$) und
G4 Vinylen (-CH = CH-) bedeutet,
und ihre Salze.

Bevorzugte erfindungsgemäße Verbindungen sind solche der Formel I, worin
R1 Wasserstoff,
R2 Methyl,
R3 Wasserstoff, Methyl, Hydroxymethyl oder Cyanomethyl,
R4 Wasserstoff,
R5 Wasserstoff,
R6 Wasserstoff,
G1 CH oder N (Stickstoff),
G2 O (Sauerstoff), NH, Methin ( = CH-) oder Methylen,
G3 Trimethylen ($-CH_2-CH_2-CH_2-$), Tetramethylen ($-CH_2-CH_2-CH_2-CH_2-$), oder Propenylen ($-CH=CH-CH_2-$) und
G4 Vinylen (-CH = CH-) bedeutet,
und ihr Salze.

Beispielhafte bevorzugte erfindungsgemäße Verbindungen können durch die folgenden Formeln Ia bis Id charakterisiert werden

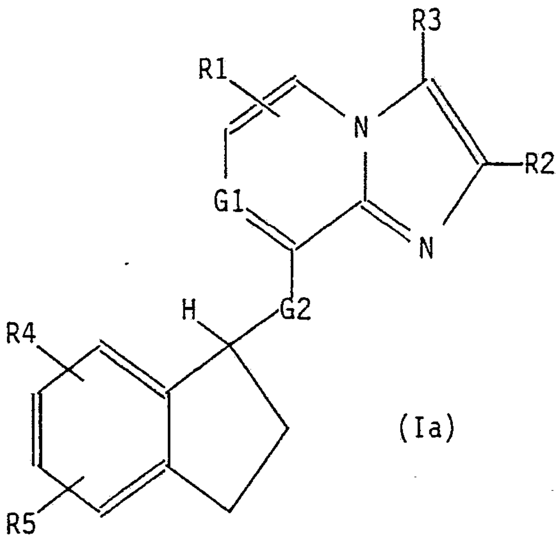

(Ia)

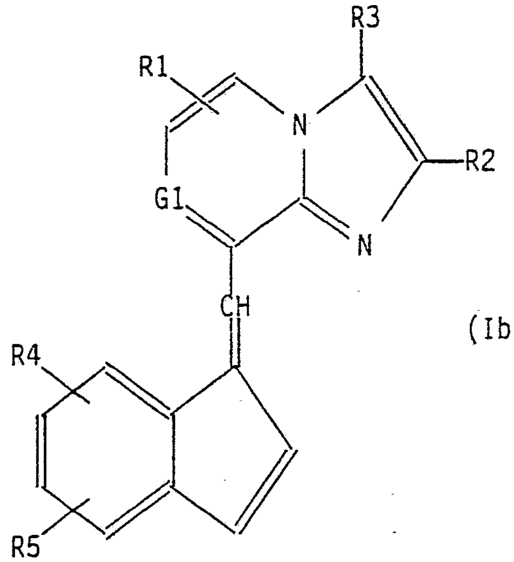

(Ib)

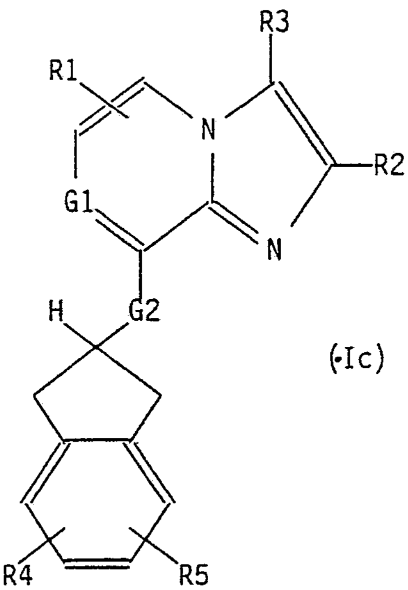

(Ic)

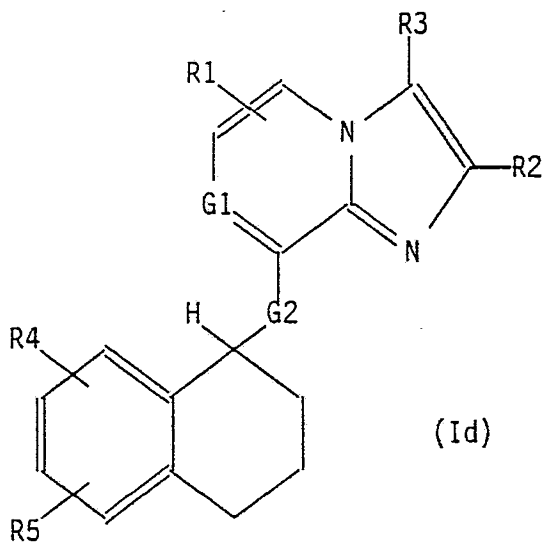

(Id)

worin die Substituenten und Symole R1, R2, R3, R4, R5 und G1 die für die bevorzugten Verbindungen der Formel I angegebenen Bedeutungen haben und G2 O (Sauerstoff), NH oder Methylen bedeutet.

Als bevorzugte erfindungsgemässe seien beispielsweise genannt:

8-(2,3-Dihydro-1-indenyloxy)-2-methyl-imidazo[1,2-a]pyridin
8-(2,3-Dihydro-1-indenyloxy)-2,3-dimethyl-imidazo[1,2-a]pyridin
3-Cyanomethyl-8-(2,3-dihydro-1-indenyloxy)-2-methyl-imidazo[1,2-a]pyridin
8-(2-Inden-1-yl-oxy)-2-methyl-imidazo[1,2-a]pyridin
8-(2-Inden-1-yl-oxy)-2,3-dimethyl-imidazo[1,2-a]pyridin
3-Cyanomethyl-8-(2-inden-1-yl-oxy)-2-methyl-imidazo[1,2-a]pyridin
2-Methyl-8-(1,2,3,4-tetrahydro-1-naphthyloxy)-imidazo[1,2-a]pyridin
2,3-Dimethyl-8-(1,2,3,4-tetrahydro-1-naphthyloxy)-imidazo[1,2-a]pyridin
3-Cyanomethyl-2-methyl-8-(1,2,3,4-tetrahydro-1-naphthyloxy)imidazo[1,2-a]pyridin
8-(2,3-Dihydro-1-indenylamino)-2-methyl-imidazo[1,2-a]pyridin
8-(2,3-Dihydro-1-indenylamino)-2,3-dimethyl-imidazo[1,2-a]pyridin
3-Cyanomethyl-8-(2,3-dihydro-1-indenylamino)-2-methyl-imidazo[1,2-a]pyridin
8-(2,3-Dihydro-1-indenylmethyl)-2-methyl-imidazo[1,2-a]pyridin
8-(2,3-Dihydro-1-indenylmethyl-2,3-dimethyl-imidazo[1,2-a]pyridin
3-Cyanomethyl-8-(2,3-dihydro-1-indenylmethyl)-2-methyl-imidazo[1,2-a]pyridin
2-Methyl-8-(1,2,3,4-tetrahydro-1-naphthylmethyl)-imidazo[1,2-a]pyridin

2,3-Dimethyl-8-(1,2,3,4-tetrahydro-1-naphthylmethyl)-imidazo[1,2-a]pyridin
3-Cyanomethyl-2-methyl-8-(1,2,3,4-tetrahydro-1-naphthylmethyl)-imidazo[1.2-a]pyridin
8-(2,3-Dihydro-1-indenyloxy)-2-methyl-imidazo[1,2-a]pyrazin
8-(2,3-Dihydro-1-indenyloxy)-2,3-dimethyl-imidazo[1,2-a]pyrazin
8-(2-Inden-1-yl-oxy)-2-methyl-imidazo[1,2-a]pyrizin
8-(2-Inden-1-yl-oxy)-2,3-dimethyl-imidazo[1,2-a]pyrazin
2-Methyl-8-(1,2,3,4-tetrahydro-1-naphthyloxy)-imidazo[1,2-a]pyrazin
2,3-Dimethyl-8-(1,2,3,4-tetrahydro-1-naphthyloxy)-imidazo[1,2-a]pyrazin
8-(2,3-Dihydro-1-indenylamino)-2-methyl-imidazo[1,2-a]pyrazin
8-(2,3-Dihydro-1-indenylamino)-2,3-diemthyl-imidazo[1,2-a]pyrazin
2-Methyl-8(1,2,3,4-tetrahydro-1-naphhylamino)-imidazo[1,2-a]pyrazin
2,3-Dimethyl-8-(1,2,3,4-tetrahydro-1-naphthylamino)-imidazo[1,2-a]pyrazin
8-(2,3-Dihydro-1-indenylmethyl)-3-hydroxymethyl-2-methyl-imidazo[1,2-a]pyrazin
8-(2,3-Dihydro-1-indenyloxy)-3-hydroxymethyl-2-methyl-imidazo[1,2-a]pyrazin
8-(2,3-Dihydro-1-indenylamino)-3-hydroxymethyl-2-methyl-imidazo[1,2-a]pyrazin
3-Hydroxymethyl-2-methyl-8-(1,2,3,4-tetrahydro-1-naphthyloxy)-imidazo[1,2-a]pyridin
3-Hydroxymethyl-2-methyl-8-(1,2,3,4-tetrahydro-1-naphthylamino)-imidazo[1,2-a]pyridin
3-Hydroxymethyl-2-methyl-8-(1,2,3,4-tetrahydro-1-naphthylmethyl)-imidazo[1,2-a]pyridin
3-Hydroxymethyl-2-methyl-8-(1,2,3,4-tetrahydro-1-napthyloxy)-imidazo[1,2-a]pyrazin
3-Hydroxymethyl-2-methyl-8-(1,2,3,4-tetrahydro-1-naphthylamino)-imidazo[1,2-a]pyrazin
3-Hydroxymethyl-2-methyl-8-(1,2,3,4-tetrahydro-1-naphthylmethyl)-imidazo[1,2-a]pyrazin
8-(2,3-Dihydro-2-indenyloxy)-3-hydroxymethyl-2-methyl-imidazo[1,2-a]pyridin
8-(2,3-Dihydro-2-indenylamino)-3-hydroxymethyl-2-methyl-imidazo[1,2-a]pyridin
8-(2,3-Dihydro-2-indenylmethyl)-3-hydroxymethyl-2-methyl-imidazo[1,2-a]pyridin
8-(2,3-Dihydro-2-indenyloxy)-3-hydroxymethyl-2-methyl-imidazo[1,2-a]pyrazin
8(2,3-Dihydro-2-indenylamino)-3-hydroxymethyl-2-methyl-imidazo[1,2-a]pyrazin
8-(2,3-Dihydro-2-indenylmethyl)-3-hydroxymethyl-2-methyl-imidazo[1,2-a]pyrazin
3-Hydroxymethyl-8-(inden-1-ylidenmethyl)-2-methyl-imidazo[1,2-a]pyridin
3-Hydroxymethyl-8-(inden-1-ylidenmethyl)-2-methyl-imidazo[1,2-a[pyrazin
2-Methyl-8-(1,2,3,4-tetrahydro-1-naphthylamino)-imidazo[1,2-a]pyridin
8-(2,3-Dihydro-2-indenylamino)-2,3-dimethyl-imidazo[1,2-a]pyridin
3-Formyl-8-(2,3-dihydro-1-indenylmethyl)-2-methyl-imidazo[1,2-a]pyridin
8-(2,3-Dihydro-1-indenylmethyl)-3-hydroxymethyl-2-methyl-imidazo[1,2-a]pyridin
2,3-Dimethyl-8-(inden-1-ylidenmethyl)-imidazo[1,2-a]pyridin
8-(Inden-1-ylidenmethyl)-2-methyl-imidazo[1,2-a]pyridin
8-(2,3-Dihydro-1-indenylmethyl)-3-dimethylaminomethyl-2-methyl-imidazo[1,2-a]pyridin
8-(2,3-Dihydro-1-indenyloxy)-3-hydroxymethyl-2-methyl-imidazo[1,2-a]pyridin
8-(2,3-Dihydro-1-indenylamino)-3-hydroxymethyl-2-methyl-imidazo[1,2-a]pyridin
und ihre Salze.

Die erfindungsgemäßen Verbindungen können über ein oder mehrere Chiralitätszentren verfügen, und zwar beispielsweise an dem Kohlenstoffatom, an dem der Rest G2 an das Ringstück G3 angeknüpft ist. Die Erfindung umfaßt daher - bei Vorliegen eines Chiralitätszentrums - sowohl die (reinen) Enantiomeren als auch deren Gemische einschließlich des Racemats. Beim Vorliegen weiterer Chiralitätszentren (z.B. wenn das Ringstück G3 durch 1-4C-Alkyl substituiert ist) umfäßt die Erfindung nicht nur die Enantiomeren, sondern auch die Diastereomeren sowie deren Gemische und Racemate.

Weiterer Gegenstand der Erfindung ist ein Verfahren zur Herstellung der erfindungsgemäßen Verbindungen und ihrer Salze. Das Verfahren ist dadurch gekennzeichnet, daß man

a) Aminoverbindungen der Forml II Carbonylverbindungen der Formel III umsetzt,

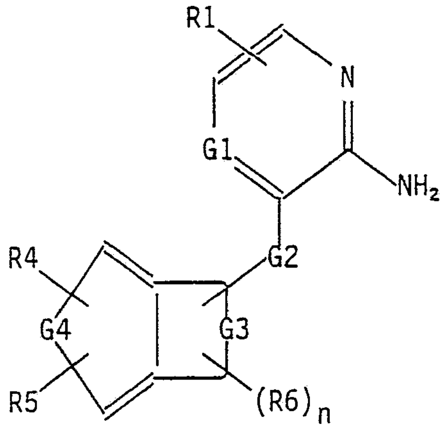 (II)

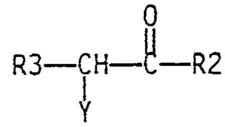 (III)

oder

b) Imidazopyridine der Formel IV mit Bicyclen der Formel V umsetzt,

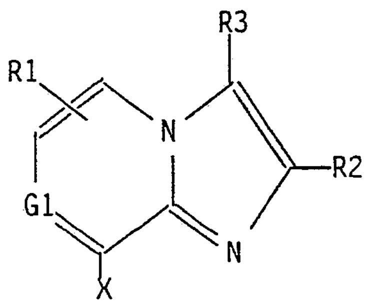 (IV)

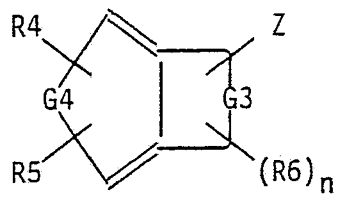 (V)

oder

c) zur Herstellung von Verbindungen der Formel I, worin R3 Formyl bedeutet, Verbindungen der Formel I, worin R3 H bedeutet, mit Dimethylformamid und Phosphoroxitrichlorid umsetzt,

oder

d) zur Herstellung von Verbindungen der Formel I, worin R3 Hydroxymethyl bedeutet, Verbindungen der Formel I, worin R3 Formyl bedeutet, hydrogeniert

oder

e) zur Herstellung von Verbindungen der Formel I, worin G2 Methylen ($-CH_2-$) bedeutet, Verbindungen der Formel I, worin G2 Methin ($=CH-$) bedeutet, hydriert,

oder

f) zur Herstellung von Verbindungen der Formel I, worin R3 Aminomethyl oder Mono-oder Di-1-4C-alkylaminomethyl bedeutet, Verbindungen der Formel I, worin R3 Wasserstoff bedeutet, mit Ammoniak oder Mono-oder Di-1-4C-alkylamin und Formaldehyd umsetzt,

oder

g) zur Herstellung von Verbindungen der Formel I, worin R3 Cyanomethyl bedeutet, Verbindungen der Formel VI

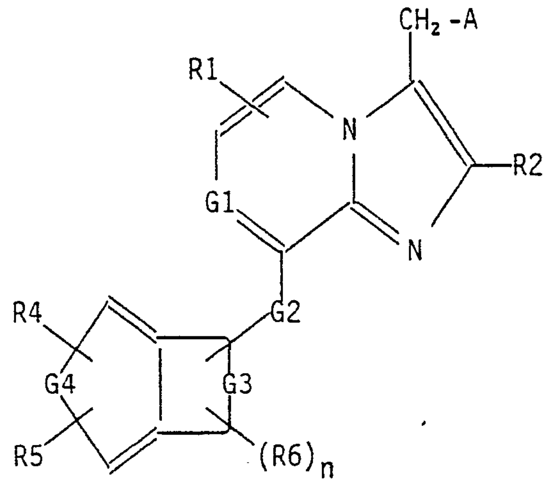

(VI)

mit Cyaniden umsetzt und gewünschtenfalls anschließend erhaltene Salze in die freien Basen oder erhaltene Basen in die Salze überführt, wobei die Ausgangsverbindungen als solche oder in Form ihrer Salze eingesetzt werden und wobei R1, R2, R3, R4, R5, R6, n, G1, G2, G3 und G4 die oben angegebenen Bedeutungen haben, Y und A geeignete Abgangsgruppen (Fluchtgruppen) darstellen und X und Z geeignete reaktive Gruppen darstellen, die unter Bildung der Gruppe G2 zu reagieren in der Lage sind.

Die Umsetzung der Aminoverbindungen II mit den Carbonylverbindungen III erfolgt in geeigneten, inerten Lösungsmitteln, beispielsweise in Alkoholen, wie Butanol, Isopropanol, Ethanol oder Methanol, oder in anderen, vorzugsweise polaren Lösungsmitteln wie Acetonitril, Dimethylformamid oder Dimethylsulfoxid. Als Reaktionstemperatur kommen insbesondere Temperaturen zwischen 50°C und 150°C in Frage, wobei die Siedetemperatur des verwendeten Lösungsmittels bevorzugt ist.

Als Abgangsgruppen Y kommen insbesondere solche Substituenten in Frage, die bei den Carbonylverbindungen III synthetisch leicht zugänglich sind und die bei der Reaktion mit den Aminoverbindungen II leicht abgespalten werden können. Als bevorzugte Abgangsgruppen Y sind vor allem Halogenatome, insbesondere Chlor und Brom zu nennen.

Die Umsetzung von II mit III erfolgt vorteilhafterweise (um nicht mit einem Überschuß an Verbindung II arbeiten zu müssen) in Gegenwart geeigneter Basen. Als solche seien Alkalihydroxide (wie Natrium-oder Kaliumhydroxid), Alkalicarbonate (wie Natrium-oder Kaliumcarbonat) oder organische Amine, bevorzugt sekundäre oder tertiäre Amine - wie Diisopropylamin oder Triethylamin - genannt.

Die Art der Umsetzung der Imidazopyridine IV mit den Bicyclen V hängt von den Strukturen der reaktiven Gruppen X und Z ab, die wiederum von der gewünschten Gruppe G2 abhängig sind. Folgende reaktive Gruppen seien in Abhängigkeit von G2 beispielsweise genannt:

| | | | |
|---|---|---|---|
| G2 = O (Sauerstoff) | | X = OH | Z = Halogen |
| | oder | X = Halogen | Z = OH |
| G2 = NH | | X = $NH_2$ | Z = Halogen |
| | oder | X = Halogen | Z = $NH_2$ |
| G2 = N-1-4C-Alkyl | | X = NH-1-4C-Alkyl | Z = Halogen |
| | oder | X = Halogen | Z = NH-1-4C-Alkyl |
| G2 = S (Schwefel) | | X = SH | Z = Halogen |
| | oder | X = Halogen | Z = SH |
| G2 = 1-4C-Alkylen | X und Z sind Gruppen, die in einer Kondensationsreaktion unter Ausbildung einer C-C-Bindung miteinander reagieren können. | | |
| G2 = Methin (=CH-) | | X = CHO | Z = $<^{H}_{H}$ |

Die Reaktion von IV mit V wird in geeigneten, inerten Lösungsmitteln durchgeführt, wobei die Kondensation unter Abspaltung von H-Halogen die Gegenwart einer geeigneten Base bzw. die vorherige Deprotenierung (z.B. mit einem Hydrid, wie Natriumhydrid) erfordert, und wobei die Knüpfung der C-C-Bindung z.B. als Aldolkondensation bzw. die Bildung der Methingruppe als Wittig-Reaktion auf eine für den Fachmann gebräuchliche Weise erfolgt.

Welche Reaktionsbedingungen für die Durchführung der Verfahrensvariante b) im einzelnen erforderlich sind, ist dem Fachmann aufgrund seines Fachwissens geläufig.

Die Umsetzung gemäß Verfahrensvariante c wird auf eine für den Fachmann gebräuchliche Weise, wie sie für die Vilsmaier-Reaktion bekannt ist, in inerten Lösungsmitteln (vorzugsweise Dimethylformamid) bei Temperaturen, die vorteilhafterweise 30°C nicht überschreiten, durchgeführt.

Die Hydrogenierung (Reduktion) gemäß Verfahrensvariante d wird mit den üblichen Reduktionsmitteln, wie sie für die Reduktion von Aldehyden zu Alkohlen gebräuchlich sind, in inerten, wasserfreien Lösungsmitteln bei mäßigen Temperaturen (z. b. Raumtemperatur) durchgeführt. Als Reduktionsmittel seien beispielsweise Lithiumaluminiumhydrid oder Natriumborhydrid genannt. Als Lösungsmittel kommen unter anderem Alkohole. z. B. Methanol oder Ethanol infrage.

Die Hydrierung gemäß Verfahrensvariante e wird auf eine für den Fachmann übliche Weise durchgeführt, beispielsweise als katalytische Hydrierung mit Palladium auf Holzkohle bei Raumtemperatur in Ethanol oder Methanol in einer Wasserstoffatmosphäre.

Die Einführung der Amino-, Monoalkylamino-bzw. Dialkylaminomethylgruppe gemäß Verfahrensvariante f erfolgt ebenfalls auf eine für den Fachmann geläufige Weise, wie sie für die Durchführung der Mannich-Reaktion mit Formaldehyd und Amin gebräuchlich ist.

Die in Verfahrensvariante g abzuspaltenden Abgangsgruppen A sind beispielsweise Halogenatome (Chlor oder Brom), oder quarternierte Aminogruppen, die durch die Nitrilgruppe auf übliche Weise substituiert werden. Als Cyanide kommen vor allem Erdalkali-oder Alkalicyanide, insbesondere Natriumcyanid oder Kaliumcyanid infrage.

Die Isolierung und Rienigung der erfindungsgemäßen Substanzen erfolgt in an sich bekannter Weise z.B. derart, daß man das Lösungsmittel im Vakuujm abdestilliert und den erhaltenen Rückstand aus einem geeigneten Lösungsmittel umkristallisiert oder einen der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylenchlorid oder Chloroform, oder ienem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nchtlösungsmittel für das Anlagerungssalz oder durch Verdampfen des Lösungsmittels gewonnen. Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Quartäre Ammoniumsalze erhält man in analoger Weise durch Auflösen der freien Base in einem geeigneten Lösungsmittel, z.B. in Aceton, oder in einem chlorierten Kohlenwasserstoff, wie Methylenchlorid oder Chloroform, dem das gewünschte Alkylierungsmittel anschließend zugegeben wird. Auch diese Salze werden durch Filtrieren, Umfällen, Ausfällen mit einem Nichtlösungsmittel für das Anlagerungssalz oder durch Verdampfen des Lösungsmittels gewonnen.

Die Ausgangsverbindungen II, III, IV, V und VI sind literaturbekannt oder können in Analogie zu literaturbekannten Methoden, beispielsweise in Analogie zu J.J.Kaminski et al. [J.Med.Chem. 28, 876) (1985)] hergestellt werden.

Die folgenden Beispiele erläutern die Erfindung näher ohne sie einzuschränken. Die in den Beispielen namentlich aufgeführten Verbindungen der allgemeinen Formel I sowie die Salze dieser Verbindungen sind bevorzugter Gegenstand der Erfindung. Schmp. bedeutet Schmelzpunkt, für Stunde(n) wird die Abkürzung h und für Minuten die Abkürzung Min. verwendet. Unter "Ether" wird Diethylether verstanden.

## Beispiele

### 1. 8-(2,3-Dihydro-1-indenyloxy)-2,3-dimethyl-imidazo[1,2-a]pyridin

Zur Lösung von 2,0 g 8-Hydroxy-2,3-dimethyl-imidazo[1,2-a]pyridin in trockenem Dimethylformamid (40 ml) werden unter einer Inertgasatmosphäre 0,24 g Natriumhydrid (85 %ig in Öl) bei Raumtemperatur zugegeben und 30 Min. gerührt. Anschließend werden 1,8 g 1-Chlor-indan zugegeben. Das heterogene Gemisch wird 4 h auf dem Ölbad bei 50°C gehalten, wobei eine klare Lösung entsteht, die im Vakuum bis zur Trockene eingeengt wird. Der Rückstand wird in wenig Wasser gelöst, dreimal mit je 40 ml Ethylacetat extrahiert und die vereinigten Extrakte nach Abziehen des Lösungsmittels an Kieselgel gereinigt (Fließmittel: Methylenchlorid/Methanol = 95:05). Nach Vereinigung der Fraktionen mit $R_f$ = 0.5 wird das Lösungsmittel abgezogen und der feste Rückstand aus Acetonitril umkristallisiert, wobei 0,6 g der Titelverbindung vom Schmp. 159-61°C erhalten werden.

### 2. 8-(2,3-Dihydro-1-indenyloxy)-1,2,3-trimethyl-imidazo[1,2-a]pyridiniumiodid

280 mg 8-(1-Indanyl)-2,3-dimethyl-imidazo[1,2-a]pyridin aus Beispiel 1 werden in einem Gemisch aus 5 ml Aceton und 0,5 ml Methanol (jeweils wasserfrei) gelöst und mit 0,12 ml Methyliodid versetzt. Der sich langsam abscheidende Niederschlag wird nach 12 h abgesaugt und mit wenig Diethylether gewaschen. Es werden 210 mg der Titelverbindung vom Schmp. 308-310°C (Zersetzung) erhalten.

### 3. 2,3-Dimethyl-8-(1,2,3,4-tetrahydro-1-naphthyloxy)-imidazo-[1,2-a]pyridin

Analog Beispiel 1 werden aus 2 g 8-Hydroxy-2,3-dimethyl-imidazo[1,2-a]-pyridin und 1,2,3,4-Tetrahydro-1-chlor-naphthalin 0,7 g der Titelverbindung als Öl erhalten.

4. 3-Cyanomethyl-8-(2,3-dihydro-1-indenyloxy)-2-methyl-imidazo[1,2-a]pyridin

Analog Beispiel 1 werden aus 0,3 g 3-Cyanomethyl-8-hydroxy-2-methyl-imidazo[1,2-a]pyridin mit 1-Chlorindan 0,15 g der Titelverbindung vom Schmp. 170-170,5°C (aus Acetonitril) erhalten.

5. 8-(2,3-Dihydro-1-indenyloxy)-2-methyl-imidazo[1,2-a]pyridin

Analog Beispiel 1 werden aus 1,6 g 8-Hydroxy-2-methyl-imidazo[1,2-a]pyridin und 1-Chlorindan 1,1 g der Titelverbindung vom Schmp. 179-181°C erhalten.

6. 8-(2,3-Dihydro-1-indenyloxy)-2-methyl-imidazo[1,2-a]pyrazin

Zu einer 0°C kalten Suspension von 0,24 g Natriumhydrid (80% in Öl) in Dimethylformamid unter einer Inertgasatmosphäre werden 0,8 g 1-Indanol und nach weiteren 30 Min. 1,00 g 8-Chlor-2-methyl-imidazo[1,2-a]pyrazin zugegeben. Nach 1 h Rühren bei 0°C wird das Lösungsmittel im Hochvakuum weitestgehen abgezogen, der Rückstand in 100 ml Phosphatpuffer (pH = 7.4) aufgenommen und mit Ethylacetat dreimal extrahiert (je 40 ml). Die vereinigten Extrakte werden über Natriumsulfat getrocknet. Das Lösungsmittel wird im Vakuum abgezogen und der verbleibende kristalline Rückstant aus Diisoptopylether/Diethylether umkristallisiert. Es werden 0,8 g der Titelverbindung vom Schmp. 132-134°C erhalten.

7. 2-Methyl-8-(1,2,3,4-tetrahydro-1-naphthyloxy)-imidazo[1,2-a]pyrazin

Analog Beispiel 6 wird aus 8-Chlor-2-methyl-imidazo[1,2-a]pyrazin und 1,2,3,4-Tetrahydro-1-naphthol die Titelverbindung vom Schmp. 151-152°C (aus Diethylether) erhalten.

8. 8-(2,3-Dihydro-1-indenylamino)-2,3-dimethyl-imidazo[1,2-a]pyridin

Eine Lösung von 1,5 g 8-Amino-2,3-dimethyl-imidazo[1,2-a]pyridin in 50 ml trockenem Dioxan wird mit 1,4 ml Triethylamin und 1,5 g 1-Chlorindan versetzt und 6 h bei 50°C gehalten. Anschließend wird in Vakuum bis zur Trockene eingeengt und der verbleibende Rückstand an Kieselgel (Fließmittel: Methylenchlorid/Methanol = 95:05) gereinigt. Nach Umkristallisation aus Diethylether werden 0,2 g der Titelverbindung vom Schmp. 141-143°C erhalten. Das Hydrochlorid der Titelverbindungung wird durch Auflösen der freien Base in einer äquimolaren Menge 0.1N Salzsäure und Entfernung des Wassers im Vakuum erhalten.

9. 2-Methyl-8-(1,2,3,4-tetrahydro-1-naphthylamino)-imidazo[1,2-a]pyridin

Die Titelverbindung wird als zähflüssiges Öl analog Beispiel 8 aus 8-Amino-2-methyl-imidazo[1,2-a]-pyridin-Hydrobromid und 1-Brom-1,2,3,4-tetrahydronapthtaline in 43 %iger Ausbeute erhalten.

10. 8-(2,3-Dihydro-2-indentylamino)-2,3-dimethyl-imidazo[1,2-a]pyridin

Die Umsetzung von 8-Amino-2,3-dimethyl-imidazo[1,2-a]pyridin mit 2-Brom-2,3-dihydroinden analog Beispiel 8 liefert die Titelverbindung als zähflüssiges Öl in 22 %iger Ausbeute.

11. 8-(2,3-Dihydro-1-indenylamino)-2-methyl-imidazo[1.2-a]pyridin

Die Titelverbindung wird als zähflüssiges Öl analog Beispiel 8 durch Umsetzung von 8-Amino-2-methyl-imidazo[1,2-a]pyridin-Hydrobromid mit 1-Chlor-2,3-dihydroinden in Dioxan unter Zugabe von überschüssigem Triethylamin durch 6-stündiges Kochen am Rückfluß und anschließende Reinigung an Kieselgel (Fließmittel: Methylenchlorid/Methanol = 95:05) in 35 %iger Ausbeute erhalten.

12. 3-Formyl-8-(2,3-dihydro-1-indenylmethyl)-2-methyl-imidazo[1,2-a]pyridin

0,28 ml Phosphoroxitrichlorid werden bei Raumtemperatur zu 2 ml Dimethylformamid so zugetropft, daß die Innentemperatur nicht über 30 °C steigt. Nach 1 h werden 0,5 g 8-(2,3-Dihydro-1-indenylmethyl)-2-methylimidazo[1,2-a]pyridin (aus Beispiel 17) gelöst in 5 ml Dimethylformamid zugetropft (15 Min.) und die Lösung noch 8 h bei 35-40°C erwärmt. Anschließend wird auf Eis gegossen, 1 h gerührt, mit 6N-Natronlauge auf pH 8-9 gestellt und nach 30 Min. Rühren bei pH 8-9 der ausgefallene Niederschlag abgesaugt und gut mit Wasser gewaschen. Der getrocknete Filterrückstand wird aus Isopropanol umkristallisiert. Es werden 0,3 g der Titelverbindung vom Schmp. 138-140°C erhalten.

13. 8-(2,3-Dihydro-1-indenylmethyl)-3-hydroxymethyl-2-methyl-imidazo[1,2-a]pyridin

Zur Suspension von 2,9 g 3-Formyl-8-(2,3-dihydro-1-indenylmethyl)-2-methyl-imidazo[1,2-a]pyridn in 15 ml Methanol werden unter kräftigem Rühren bei Raumtemperatur 0,24 g käufliches 97 %iges Nastriumborhydrid gegeben und 4 h nachge rührt. Anschließend werden 20 ml Wasser zugesetzt, die Lösung auf pH 9 mittels 2N-Natronlauge e3ingestellt, mit Methylenchlorid extrahiert (3x30 ml) die vereinigten Extrakte über Natriumsulfat getrocknet und im Vakuum das Lösungsmittel abgezogen. Der Rückstand wird aus Isopropanol umkristallisiert und ergibt 0,3 g der Titelverbindung vom Schmp. 176-177°C.

14. 2,3-Dimethyl-8-(inden-1-ylidenmethyl)-imidazo[1,2-a]pyridin

In einer frisch zubereiteten Natriummethylat-Lösung (4,0 g N in 180 ml trockenem Methanol) werden 20,0 g destilliertes Inden gelöst und bei 50°C Ölbadtemperatur 3,0 g 8-Formyl-2,3-dimethyl-imidazo[1,2-a]-pyridin eingetragen. Nach 30 Min. wird das Lösungsmittel im Vakuum abgezogen, der Rückstand in 50 ml 5N-Salzsäure aufgenommen und überschüssiges Inden mit Diethylether extraktiv entfernt. Die wässrige Phase wird anschließend neutralisiert und mit Ethylacetat extrahiert (3x50 ml), das Lösungsmittel abgezogen und der Rückstand an Kieselgel gereinigt (Fließmittelgemisch: Methylenchlorid/Methanol = 98:02). Nach Umkristallisation aus Diisopropylether werden 3,5 g der Titelverbindung vom Schmp. 104-105°C erhalten.

15. 8-(Inden-1-ylidenmethyl)-2-methyl-imidazo[1,2-a]pyridin

Die Titelverbindung wird analog Beispiel 14 durch Umsetzung von Inden mit 8-Formyl-2-methyl-imidazo[1,2-a]pyridin erhalten. Schmp. nach Umkristallisation aus Acetonitril: 168-169°C.

16. 2,3-Dimethyl-8-(2,3-dihydro-1-indenylmethyl)-imidazo[1,2-a]pyridin

1,5 g 2,3-Dimethyl-8-(inden-1-ylidenmethyl)-imidazo[1,2-a]pyridin (aus Beispiel 14), gelöst in 200 ml Methanol, werdin unter geringem Druck an einem käuflichen Pd/C-Katalysator (0,1 g) bei Raumtemperatur hydrogeniert (5 h). Danach wird der Katalysator abfiltriert, das Filtrat zur Trockene im Vakuum eingeengt und der feste Rückstand aus Petrolether umkristallisiert. Es werden 0,9 g der Titelverbindung vom Schmp. 82-84°C erhalten. Das Hydrochlorid schmilzt bei 195 - 200°C (Zers.)

17. 8-2,3-Dihydro-1-indenylmethyl)-2-methyl-imidazo[1,2-a]pyridin

Die Titelverbindung vom Schmp. 96-97°C (Diisopropylether) wird analog Beispiel 16 durch Hydrogenierung von 8-(Inden-1-ylidenmethyl)-2-methyl-imidazo[1,2-a]pyridin an Pd/C erhalten.

18. 8-(2,3-Dihydro-1-indenylmethyl)-3-dimethylaminomethyl-2-methyl-imidazo[1,2-a]pyridin

Ein Gemisch aus 8-(2,3-Dihydro-1-indenylmethyl)-2-methyl-imidazo[1,2-a]pyridin (1,3 g), Dimethylamin-Hydrochlorid (0,85 g), Paraformaldehyd (0,32 g) und Methanol (10 ml) wird 5 h am Rückfluß erhitzt und die entstandene Lösung nach Abkühlung auf Raumtemperatur mit konzentrierter Salzsäure (10 ml) versetzt. Nach dem Rühren über Nacht bei Raumtemperatur wird der ausgefallene Niederschlag abgesungt, in wenig Wasser gelöst, mit Natriumhydrogencarbonat schwach alkalisch gestellt und mit Methylenchlorid (3x50 ml) extrahiert. Die vereinigten Extrakte werden über Kaliumcarbonat getrocknet und das Lösungsmittel im Vakuum abgezogen. Die Titelverbindung, die als halbfester Rückstand anfällt, wird ohne weitere Reinigung in Beispiel 19 weiterverwendet.

19. 3-Cyanomethyl-8-(2,3-dihydro-1-indenylmethyl)-2-methyl-imidazo[1,2-a]pyridin

Zu 0,8 g 8-(2,3-Dihydro-1-indenylmethyl)-3-dimethylaminomethyl-2-methyl-imidazo[1,2-a]pyridin (aus Beispiel 18), gelöst in 10 ml Ethanol, werden bei Raumtemperatur inter Rühren 0,4 ml Methyliodid zugetropft (10 Min.). Nach 15 h wird der ausgefallene Niederschlag abfiltriert, mit wenig kaltem Ethanol gewaschen und im Vakuum getrocknet. Auflösung in 10 ml Dimethylformamid und Zugabe von 0,6 Natriumcyanid zu dieser Lösung ergibt nach 2-stündigem Erhitzen bei 100°C und Reinigung an Kieselgel (Fließmittel: Methylenchlorid/Methanol = 95:05) die Titelverbindung in 37 %iger Ausbeute als bräunliches, zähflüssiges Öl.

20. 8-Hydroxy-3-hydroxymethyl-2-methyl-imidazo[1,2-a]pyridin

4,6 g 8-Benzyloxy-3-hydroxymethyl-2-methyl-imidazo[1,2-a]pyridin gelöst in 260 ml Methanol werden bei Raumtemperatur über Pd/C mit Wasserstoff über 1,5 h gesättigt. Anschließend wird der Katalysator abfilteriert, das Filtrat zur Trockene im Vakuum eingeengt und der Rückstand aus einem Ethanol/Methanol = 2:3-Gemisch umkristallisiert, wobei 2,4 g der Titelverbindung vom Schmp. 327-329°C erhalten werden.

21. 8-(2,3-Dihydro-1-indenyloxy)-3-hydroxymethyl-2-methyl-imidazo[1,2-a]pyridin

Zur Suspension von 110 mg käuflichem Natriumhydrid (80 %ig in Paraffinöl) in 20 ml trockenem Dimethylformamid werden bei 0°C unter einer Inertgasatmosphäre 600 mg 8-Hydroxy-3-hydroxymethyl-20methyl-imidazo[1,2-a]pyridin in kleinen Portionen eingetragen, die Temperatur 30 Min. auf Raumtemperatur erhöht und 790 mg 1-Bromindan gelöst in 20 ml Dimethylformamid zugetropft. Man rührt noch eine

weitere Stunde, verdünnt mit 200 ml Eiswasser, extrahiert mit Ethylacetat, trocknet die vereinigten Extrakte über Kaliumcarbonat und engt im Vakuum bis zur Trockene ein. Das verbleibende zähflüssige Öl wird an Kieselgel (Fließmittelgemisch: Methylenchlorid/Methanol = 99:01) gereinigt. Es werden 230 mg der Titelverbindung vom Schmp. 150-152°C (aus Methanol) erhalten. Das Hydrochlorid schmilzt bei 174 - 175°C (aus Isopropanol).

22. 8-Amino-3-hydroxymethyl-2-methyl-imidazo[1,2-a]pyridin

Eine Lösung von 3,8 g 3-Hydroxymethyl-20methyl-8-nitro-imidazo[1,2-a]pyridin in 300 ml Methanol wird bei Raumtemperatur über einer katalytischen Menge Pd/C mit Wasserstoff gesättigt. Nach 2 h wird der Katalysator abfiltriert und das Filtrat im Vakuum bis zur Trockene eingeengt. Das so erhaltene Produkt wird ohne weitere Reinigung für die nächste Stufe verwendet.

23. 8-(2,3-Dihydro-1-indenylamino)-3-hydroxymethyl-2-methyl-imidazo[1,2-a]pyridin

Zu einer Lösung von 0,6 g 8-Amino-3-hydroxymethyl-2-methyl-imidazo[1,2-a]pyridin in 20 ml Dioxan werden 0,78 g 1-Brom-2,3,-dihydroinden gelöst in 5 ml Dioxan zugetropft. Nach 2 h Rühren bei Ramtemperatur wird mit Natriumhydrogencarbonat-Lösung die zuvor mit 100 ml Eiwasser verdünnte Reaktionslösung auf pH 8 eingestellt und mit Ethylacetat extrahiert. Das nach dem Abdampfen des Ethylacetats erhaltene zähflüssige dunkle Öl wird an Kieselgel (Fließmittelgemisch: Methylenchlorid/Methanol = 95:05) gereinigt, wobei 0,15 g der Titelverbindung als zähes Öl erhalten werden.

24. 8-Amino-2,3-dimethyl(-imidazo[1,2-a]pyridin

Die Titelverbinduing vom Schmp. 148 - 150°C (Methanol) wird analog Beispiel 22 aus 2,3-Dimethyl-8-nitro-imidazo[1,2-a]pyridin erhalten.

Gewerbliche Anwendbarkeit

Die Verbindungen der Formel I und ihre Salze besitzen wertvolle pharmakologische Eigenschaften, die sie gewerblich verwertbar machen. Sie weisen insbesondere eine ausgezeichnete Magen-und Darmschutzwirkung bei Warmblütern auf. Darüberhinaus zeichnen sich die erfindungsgemäßen Verbindungen durch das Fehlen wesentlicher Nebenwirkungen und eine große therapeutische Breite aus.

Unter "Magen-und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. bestimmte Antiphlogistika und Antirheumatika), Chemikalien (z.B. Ethanol), Magensäure oder Streßsituationen verursacht werden können.

In ihren ausgezeichneten Eigenschaften erweisen sich die erfindungsgemäßen Verbindungen an verschiedenen in-vitro-und in-vivo-Modellen überraschenderweise den aus dem Stand der Technik bekannten Verbindungen deutlich überlegen. Aufgrund dieser Eigenschaften sind die Verbindungen der Formel I ihre pharmakologisch verträglichen Salze für den Einsatz in der Human-und Veterinärmedizin hervorragend geeignet, wobei sie insbesondere zur Behandlung und/oder Prophylaxe von Ulcuserkrankunkgen des Magens und/oder Darms verwendet werden.

Eine weiterer Gegenstand der Erfindung sind daher die erfindungsgemäßen Verbindungen zur Anwendung bei der Behandlung und/oder Prophylaxe der vorstehend genannten Krankheiten.

Ebenso umfaßt die Erfindung die Verwendung der erfindungsgemäßen Verbindungen zur Herstellung von Arneimitteln, die zur Behandlung und/oder Prophylaxe der vorstehend genannten Krankheiten eingesetzt werden.

Ein weiterer Gegenstand der Erfindung sind Arzneimittel, die ein oder mehrere Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfs-oder Trägerstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95% beträgt.

Welche Hilfs-bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erweisen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzelgaben zur Etzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; Tranquilizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

Hervorzuheben ist in diesem Zusammenhang insbesondere die Kombination der erfindngsgemäßen Verbindungen mit Pharmaka, die die Säuresekretion hemmen, wie beispielsweise $H_2$-Blockern (z.B. Cimetidin, Ranitidin), oder ferner mit sogenannten peripheren Anticholinergika (z.B. Pirenzepin, Telenzepin, Zolenzepin) mit dem Ziel, die Hauptwirkung in additivem oder überadditivem Sinn zu verstärken und/oder die Nebenwirkungen zu eliminieren oder zu verringern.

## Pharmakoligie

Die ausgezeichnete Magenschutzwirkung und die magnesekretionschemmende Wirkung der erfindungsgemäßen Verbindungen kann in Untersuchungen an tierexperimentellen Modellen nachgewiesen werden. Die in der nachstehend aufgeführten Tabelle untersuchten erfindungsgemäßen Verbindungen sind mit Nummern versehen worden, die den Nummern der Beispiele entsprechen.

### Prüfung der antiulcerogenen und sekretionshemmenden Wirkung an der modifizierten Shay-Ratte

In der folgenden Tabelle 1 ist der Einfluß der erfindungsgemäßen Verbindungen nach intravenöser (i. v.) Gabe auf die Läsionsbildung sowie die Säureausscheidung bei der modifizierten Shay-Ratte dargestellt.

Tabelle 1

| Lfd. Nr. | N (Anzahl der Tiere) | Dosis (µmol/kg) i.v. | Magenschutzwirkung: Minderung des Läsionsindexes (%) | Magenschutzwirkung: ca. ED50+) (µmol/kg) i.v. | Säuresekretionshemmung: Verringerung der HCl-Sekretion (%) | Säuresekretionshemmung: ca. ED50+) (µmol/kg) i.v. |
|---|---|---|---|---|---|---|
| 1 | 15 | 0,1 | 40 | | 19 | |
| | 15 | 0,3 | 58 | | 21 | |
| | 8 | 1,0 | 75 | 0,20 | 40 | 1,93 |
| | 8 | 3,0 | 100 | | 62 | |
| | 8 | 10,0 | 100 | | 78 | |
| 4 | 8 | 0,03 | 38 | | 3 | |
| | 7 | 0,1 | 47 | | -10 | |
| | 8 | 0,3 | 64 | 0,10 | 26 | 1,34 |
| | 16 | 1,0 | 86 | | 27 | |
| | 8 | 3,0 | 100 | | 83 | |

+) ED50 = Dosis (interpoliert), die den Läsionsindex bzw. die HCl-Sekretion des Rattenmagens bei der behandelten Gruppe gegenüber der Kontrollgruppe um 50 % mindert.

Methodik

Die Ulcusprovokation erfolgt an 24 Stunden nüchternen Ratten (weiblich, 180-200 g, 4 Tiere je Käfig auf hohem Gitterrost) durch Pylorusligatur (unter Diethylethernarkose) und orale Applikation von 100 mg/10ml/kg Acetylsalicylsäure. Die zu prüfenden Substanzen werden intravenös (1 ml/kg) unmittelbar nach der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens.

Der Magen wird längs der großen Kurvatur eröffnet und auf einer Korkplatte aufgespannt, nachdem zuvor die Menge des sezernierten Magensaftes (Volumen) und später sein HCl-Gehalt (Titration mit Natronlauge) bestimmt wird. Mit einem Stereomikroskop werden bei 10-facher Vergrößerung Anzahl und Größe ( = Durch-messer) vorhandener Ulcera ermittelt. Das Produkt aus Schweregrad (gemäß nachfolgender Punkteskala) und Anzahl der Ulcera dient als individueller Läsionsindex.

| Punkteskala: | |
|---|---|
| keine Ulcera | 0 |
| Ulcusdurchmesser 0,1 - 1,4 mm | 1 |
| 1,5 - 2,4 mm | 2 |
| 2,5 - 3,4 mm | 3 |
| 3,5 - 4,4 mm | 4 |
| 4,5 - 5,4 mm | 5 |
| ⟩ 5,5 mm | 6 |

Als Maß für den antiulcerogenen Effekt dient die Minderung des mittleren Läsionsindex jeder behandelten Gruppe gegenüber dem der Kontrollgruppe (=100 %). Die ED50 bezeichnet diejenige Dosis, die den mittleren Lösionsindex bzw. die HCl-Sektetion gegenüber der Kontrolle um 50 % mindert.

## Ansprüche

1. Imidazolverbindungen der Formel I

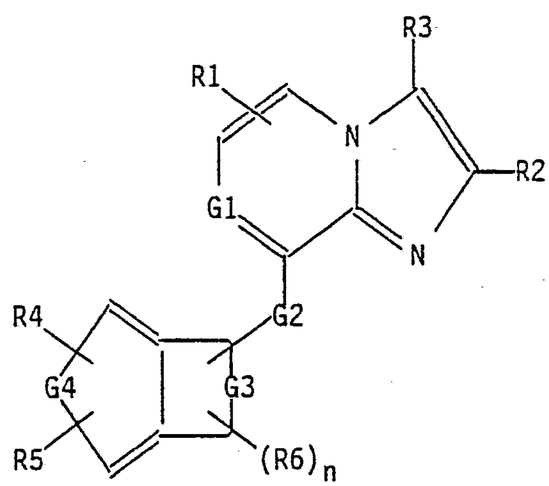

(I)

worin
R1 Wasserstoff (H), Halogen, 1-4C-Alkyl, 1-4C-Alkoxy, 1-4C-Alkoxycarbonyl oder Cyan,
R2 Wasserstoff (H), 1-4C-Alkyl, Hydroxy-1-4C-alkyl, Halo-1-4C-alkyl, Cyano1-4C-alkyl oder 1-4C-Alkoxycarbonyl,
R3 Wasserstoff (H), 1-4C-Alkyl, Formyl, Hydroxy-1-4C-alkyl, Halo-1-4C-alkyl, Cyano-1-4C-alkyl, Amino-1-4C-alkyl, Mono-oder Di-1-4C-alkylamino-1-4C-alkyl, Nitroso, Nitro, Amino, Mono-oder Di-1-4C-alkylamino oder 1-4C-Alkoxycarbonyl,
R4 Wasserstoff (H), 1-4C-Alkyl, 1-4C-Alkoxy, Halogen oder Trifluormethyl,
R5 Wasserstoff (H), 1-4C-Alkyl, 1-4C-Alkoxy, Halogen oder Trifluormethyl,
R6 Wasserstoff oder 1-4C-Alkyl,
n die Zahlen 1 oder 2,
G1 CH oder N (Stickstoff),
G2 O (Sauerstoff), NH, N-1-4C-Alkyl, S (Schwefel), Methin (=CH-) oder 1-4C-. Alkylen,
G3 Ethylen ($-CH_2CH_2-$), Trimethylen ($-CH_2-CH_2-CH_2-$), Tetramethylen ($-CH_2CH_2CH_2CH_2-$), Pentamethylen ($-CH_2CH_2CH_2CH_2CH_2-$), Propenylen ($-CH=CH-CH_2-$), 1-Butenylen ($-CH=CH-CH_2-CH_2-$) oder 2-Butylen-($-CH_2-CH=CH-CH_2-$) und
G4 S (Schwefel), O (Sauerstoff) oder Vinylen ($-CH=CH-$) bedeutet, und ihre Salze.

2. Verbindungen der Formel I nach Anspruch 1, worin
R1 Wasserstoff, Chlor oder Fluor,
R2 Wasserstoff, Methyl, Ethyl, Hydroxymethyl, Chlormethyl, Cyanomethyl, Methoxycarbonyl oder Ethoxycarbonyl,
R3 Wasserstoff, Methyl, Formyl, Hydroxymethyl, Chlormethyl, Cyanomethyl, Dimethylaminomethyl, Dimethylaminoethyl, Amino, Methoxycarbonyl oder Ethoxycarbonyl,
R4 Wasserstoff, Methyl, Methoxy, Chlor, Fluor oder Trifluormethyl,
R5 Wasserstoff,
R6 Wasserstoff oder Methyl,
n die Zahlen 1 oder 2,
G1 CH oder N (Stickstoff),
G2 O (Sauerstoff), NH, Methin (=CH-) oder Methylen,
G3 Trimethylen ($-CH_2-CH_2-CH_2-$), Tetramethylen ($-CH_2CH_2CH_2CH_2-$), Pentamethylen ($-CH_2CH_2CH_2CH_2CH_2-$), Propenylen ($-CH=CH-CH_2-$), 1-Butenylen ($-CH=CH-CH_2-CH_2-$) oder 2-Butylen ($-CH_2-CH=CH-CH_2-$) und
G4 O (Sauerstoff), S (Schwefel) oder Vinylen ($-CH=CH-$) bedeutet, und ihre Salze.

3. Verbindungen der Formel I Anspruch 1,
worin
R1 Wasserstoff, Chlor oder Fluor,
R2 Wasserstoff, Methyl oder Hydroxymethyl,
R3 Wasserstoff, Methyl, Formyl, Hydroxymethyl, Chlormethyl, Cyanomethyl oder Dimethylaminomethyl
R4 Wasserstoff, Methyl, Chlor oder Fluor
R5 Wasserstoff,
R6 Wasserstoff
G1 CH oder N (Stickstoff),
G2 O (Sauerstoff), NH, Methin (=CH-) oder Methylen,
G3 Trimethylen ($-CH_2-CH_2-CH_2-$), Tetramethylen ($-CH_2-CH_2CH_2CH_2-$) oder Propenylen ($-CH=CH-CH_2-$) und
G4 O (Sauerstoff), S (Schwefel) oder Vinylen ($-CH=CH-$) bedeutet, und ihre Salze.

4. Verbinngen der Formel I nach Anspruch 1,
worin
R1 Wasserstoff,
R2 Methyl,
R3 Wasserstoff, Methyl, Formyl, Hydroxymethyl, Cyanomethyl oder Dimethylaminomethyl,
R4 Wasserstoff,
R5 Wasserstoff,
R6 Wasserstoff,
G1 CH oder N (Stickstoff),
G2 O (Sauerstoff), NH, Methin (=CH-) oder Methylen,
G3 Trimethylen ($-CH_2-CH_2-CH_2-$), Tetramethylen ($-CH_2-CH_2-CH_2-CH_2-$), oder Propenylen ($-CH=CH-CH_2-$) und
G4 Vinylen ($-CH=CH-$) bedeutet, und ihre Salze.

5. Verbindungen der Formel I nach Anspruch 1,
worin
R1 Wasserstoff,
R2 Methyl,
R3 Wasserstoff, Methyl, Hydroxymethyl oder Cyanomethyl,
R4 Wasserstoff,
R5 Wasserstoff,
R6 Wasserstoff,
G1 CH oder N (Stickstoff),
G2 O (Sauerstoff), NH, Methin (=CH-) oder Methylen,
G3 Trimethylen ($-CH_2-CH_2-CH_2-$), Tetramethylen ($-CH_2-CH_2-CH_2-CH_2-$), oder Propenylen ($-CH=CH-CH_2-$) und
G4 Vinylen ($-CH=CH-$) bedeutet,
und ihre Salze.

6. Verbindungen nach Anspruch 5, worin R3 Hydroxymethyl bedeutet.

7. Verfahren zur Herstellung der Verbindungen der Formel I nach Anspruch 1 und ihrer Salze, dadurch gekennzeichnet, daß man

a) Aminoverbindungen der Formel II mit Carbonylverbindungen der Formel III umsetzt,

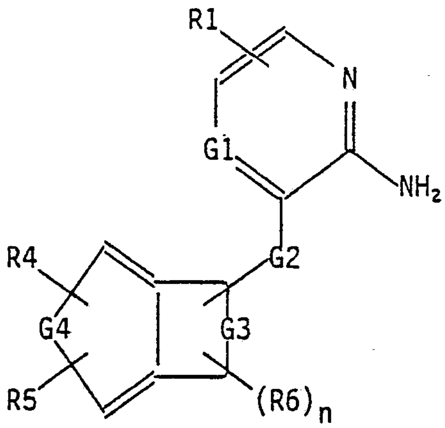 (II)

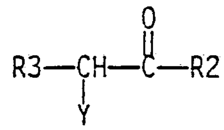 (III)

oder

b) Imidazopyridine der Formel IV mit Bicyclen der Formel V umsetzt,

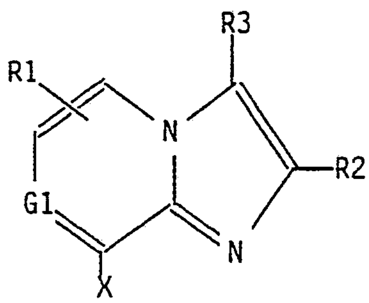 (IV)

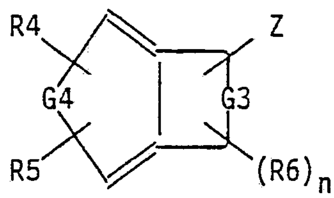 (V)

oder

c) zur Herstellung von Verbindungen der Formel I, worin R3 Formyl bedeutet, Verbindungen der Formel I, worin R3 H bedeutet, mit Dimethylformamid und Phosphoroxitrichlorid umsetzt,

oder

d) zur Herstellung von Verbindungen der Formel I, worin R3 Hydroxymethyl bedeutet, Verbindungen der Formel I, worin R3 bedeutet, hydrogeniert oder

e) zur Herstellung von Verbindungen der Formel I, worin G2 Methylen ($-CH_2-$) bedeutet, Verbindungen der Formel I, worin G2 Methin ($=CH-$) bedeutet, hydriert, oder

f) zur Herstellung von Verbindungen der Formel I, worin R3 Aminomethyl oder Mono-oder Di-1-4C-alkylaminomethyl bedeutet, Verbindungen der Formel I, worin R3 Wasserstoff bedeutet, mit Ammoniak oder Mono-oder Di-1-4C-alkylamin und Formalkehyd umsetzt,

oder

g) zur Herstellung von Verbindungen der Formel I, worin R3 Cyanomethyl bedeutet, Verbindungen der Formel VI

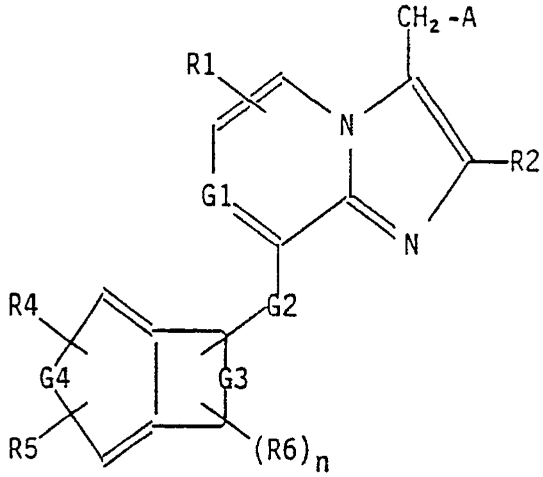

(VI)

mit Cyaniden umsetzt

umd gewünschtenfalls anschließend erhaltene Salze in die freien Basen oder erhaltene Basen in die Slaze überführt, wobei die Ausgangsverbindungen als solche oder in Form ihrer Salze eingesetzt werden und wobei R1, R2, R3, R4, R5, R6, n, G1, G2, G3, und G4 die in Anspruch 1 angegebenen Bedeutungen haben, Y und A geeignete Abgangsgruppen (Fluchtgruppen) dargestellen und X und Z geeignete reaktive Gruppen darstellen, die Bildung der Fruppe G2 zu reagieren in der Lage sind.

8. Verfahren nach Anspruch 7, dadurch gekennzeichnet, daß man eine Verbindung gemäß Anspruch 2 oder 3 oder 5 oder 6 herstellt.

9. Arzneimittel enthaltend eine oder mehrere Verbindungen nach einem oder mehreren der Ansprüche 1 bis 6 und/oder ihre pharmakologisch verträglichen Salze.

10. Verbindungen nach einem oder mehreren der Ansprüche 1 bis 6 und ihre pharmakologisch verträglichern Salze zur Anwendung bei der Verhütung und Behandlung gastrointestinaler Krankheiten.